# EUROPEAN PATENT SPECIFICATION

(11) **EP 1 088 094 B1**
(45) Date of publication and mention of the grant of the patent: **17.05.2006**
(21) Application number: 98937507.6
(22) Date of filing: 18.06.1998
(51) Int. Cl.: C12P 17/06, C07D 311/30, C07D 311/62, C09K 15/06, C12R 1/785

(54) **BIOCATALYTIC PROCESS FOR THE PREPARATION OF 3-0-ACYL-FLAVONOIDS**
BIOKATALYTISCHES VERFAHREN FÜR DIE HERSTELLUNG VON 3-O-ACYL-FLAVONOIDEN
PROCEDE BIOCATALYTIQUE DE PREPARATION DE 3-O-ACYL-FLAVONOIDES

(43) Date of publication of application: 04.04.2001
(73) Proprietor: CONSIGLIO NAZIONALE DELLE RICERCHE, 00185 Roma (IT); Rao-Erbe di Rao Felice, 95027 S. Gregorio (IT)
(72) Inventor: NICOLOSI, Giovanni, Ist. per Studio delle Sost., Via del Santuario, 110, 95028 Valverde (IT); PIATTELLI, Mario, Inst. per lo Studio delle Sost., Via del Santuario, 110, 95028 Valverde (IT); LAMBUSTA, Daniela, Ist. per Studio delle Sostanze, Via del Santuario, 110, 95028 Valverde (IT); PATTI, Angela, Inst. per lo Studio delle Sostanze, Via del Santuario, 110, 95028 Valverde (IT)
(74) Representative: Minoja, Fabrizio
(86) International application number: PCT/EP1998/003736
(87) International publication number: WO 1999/066062

(56) References cited:
- EP-A- 0 618 203
- CHEMICAL ABSTRACTS, vol. 114, no. 11, 18 March 1991 Columbus, Ohio, US; abstract no. 101429, NATOLI, MARIAPINA ET AL: "Enzyme-catalyzed alcoholysis of flavone acetates in organic solvent" XP002093460 & TETRAHEDRON LETT. (1990), 31(50), 7371-4 CODEN: TELEAY;ISSN: 0040-4039,1990,
- CHEMICAL ABSTRACTS, vol. 124, no. 5, 29 January 1996 Columbus, Ohio, US; abstract no. 55331, OZEGOWSKI, RUEDIGER ET AL: "Enzymes in organic synthesis. 25. Lipase-catalyzed sequential esterification of (.+-.)-2-methylbutanedioic anhydride - a biocatalytical access to an enantiomerically pure 1- monoester of (S)-2-methylbutanedioic acid" XP002093461 & LIEBIGS ANN. (1995), (9), 1699-702 CODEN: LANAEM;ISSN: 0947-3440,1995,
- LAMBUSTA, D. ET AL.: "Enzyme-Mediated Regioprotection-Deprotection of Hydroxyl-Groups in (+)-Catechin." SYNTHESIS, vol. 11, 1993, pages 1155-1158, XP002093458 cited in the application
- NATOLI, M. ET AL.: "Regioselective Alcoholysis of Flavonoid Acetates with Lipase in an Organic Solvent." J. ORG. CHEM., vol. 57, no. 21, 1992, pages 5776-5778, XP002093459 cited in the application
- CHEMICAL ABSTRACTS, vol. 99, no. 25, 19 December 1983 Columbus, Ohio, US; abstract no. 207106, SANSEI PHARMACEUTICAL CO. LTD., JAPAN: "Esters of fatty acids with quercetin as skin-whitening cosmetics" XP002093462 & JP 58 131911 A (SANSEI PHARMACEUTICAL CO. LTD., JAPAN)
- CHEMICAL ABSTRACTS, vol. 105, no. 1, 7 July 1986 Columbus, Ohio, US; abstract no. 3525, KULANTHAIVEL, PALANIAPPAN ET AL: "A new truxillate and some flavonoid esters from the leaf gum of Traversia baccharoides Hook f" XP002093463 & CAN. J. CHEM. (1986), 64(3), 514-19 CODEN: CJCHAG;ISSN: 0008-4042,1986,
- CHEMICAL ABSTRACTS, vol. 105, no. 19, 10 November 1986 Columbus, Ohio, US; abstract no. 166411, PERRISSOUD, D. ET AL: "Inhibiting or potentiating effects of flavonoids on carbon tetrachloride-induced toxicity in isolated rat hepatocytes" XP002093464 & ARZNEIM.-FORSCH. (1986), 36(8), 1249-53 CODEN: ARZNAD;ISSN: 0004-4172,1986,
- CHEMICAL ABSTRACTS, vol. 125, no. 25, 16 December 1996 Columbus, Ohio, US; abstract no. 320063, NANJO, FUMIO ET AL: "Scavenging effects of tea catechins and their derivatives on 1,1-diphenyl-2-picrylhydrazyl radical" XP002093465 & FREE RADICAL BIOL. MED. (1996), 21(6), 895-902 CODEN: FRBMEH;ISSN: 0891-5849, 1996,
- CHEMICAL ABSTRACTS, vol. 120, no. 15, 11 April 1994 Columbus, Ohio, US; abstract no. 191378, TOBIASON, FRED L.: "MNDO and AM1 molecular orbital and molecular mechanics analyses of (+)-catechin, (-)-epicatechin, and their 3-O-acetyl derivatives" XP002093466 & BASIC LIFE SCI. (1992), 59(PLANT POLYPHENOLS), 459-78 CODEN: BLFSBY;ISSN: 0090-5542,1992,
- CHEMICAL ABSTRACTS, vol. 91, no. 21, 19 November 1979 Columbus, Ohio, US; abstract no. 175201, ZYMA S. A., SWITZ.: "O-Substituted (+)-cyanidan-3-ols" XP002093467 & JP 54 081274 A (ZYMA S. A., SWITZ.)

## Description

Flavonoids are an important class of natural polyphenols present in various plants.

Many of them possess important biological activities, such as hepatoprotective, anticholesterolemic, antineoplastic, antiinflammatory, antiinfluenza, antiulcer, vasoprotective. Some flavonoids exploit inhibiting activity against tyrosine kinase and phosphatidyl-inositol kinase. Moreover, their antioxidant properties against peroxide radicals are comparable, if not greater, than conventional phenolic antioxidants. Many of these chemical and biological properties are strictly correlated to the position of hydroxyl groups on the flavane skeleton.

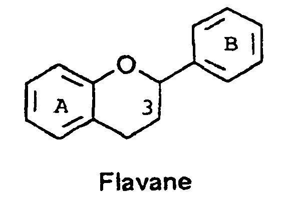

Due to their polyhydroxylated nature flavonoids are insoluble in lipophilic media (oils and emulsions) and weakly bioavailable, so that their employment is limited.

It is necessary to develop a synthetic process to obtain selective esterification of hydroxyl group at the C-3 position, without involving the other phenolic groups in the molecule.

Regioeselective alcoholysis of peracetylated flavonoids have been reported by Natoli et al. (Natoli, M; Nicolosi, G.; Piattelli, M, *J. Org. Chem.* 1992, 57, 5776-5778). The process described make use of *Pseudomonas cepacia* lipase, which catalysed the alcoholysis of the acetoxyl group in different position, but not at C-3; nevertheless the 3-acetoxyl derivative is recovered in very low yield.

Lambusta e al., *Synthesis* 1993, 1155-1158 reported the preparation of (+)-3-O-acetylcatechin by alcoholysis of peracetylated (+)-catechin in the presence of *Pseudomonas cepacia* lipase, but the reaction conditions adopted are detrimental to the enzyme and consequently its fast inactivation is observed.

EP 0618203 reports catechins acylated at position C-3, prepared by esterifications of free catechin catalysed by *Streptomyces rachei* o *Aspergillus niger* carboxylesterase. Using this process authors enables for esters with acetyl, propyl and butyryl groups.

### Summary of the Invention

It has now been found that it is possible to obtain 3-monoesters of flavonoid as the only reaction product by carrying out the alcoholysis of a peracylated flavonoid in organic solvent in the presence of *Mucor miehei* lipase.

Therefore, it is an an object of the present invention an efficient method of producing 3-monoesters of flavonoids, comprising:
a) chemical esterification of the flavonoid with an aliphatic acyl group having from 1 to 18 carbon atoms, to the corresponding peracylated flavonoid, or alternatively partially acylated flavonoid;
b) alcoholysis of the above ester with an aliphatic alcohol having from 1 to 8 carbon atoms or with a polyol in the presence of *Mucor miehei* lipase in an organic solvent.

Advantageously, according to the process of the present invention, the lipase retains its catalytic activity for more process cycles*

This invention also comprises new monoesters of flavonoids.

### Detailed description of the invention

The lipase from *Mucor miehei* is well known since long time, but has never been employed in the esterification of phenols or hydrolysis of peracylated phenols.

According to the present invention, the alcoholysis of flavonoids, partially or exhaustively acylated, is carried out in an organic solvent.

Preferred examples of solvent can be aliphatic and aromatic hydrocarbons, halogenated hydrocarbons, ethers, more preferably terbutyl methyl ether.

All the flavonoids possessing a hydroxyl function at the C-3 position can be used. Examples of such flavonoids are: quercetin, galangin, morin, fisetin, kampferolo, kampferide, (+)-catechin, (-)-catechin, (+)-epicatechin, (-)-epicatechin.

In a preferred embodiment, the process of the present invention provides the use of the lipase from *Mucor miehei* adsorbed on solid support, such as the commercially available Lipozyme® IM from Novo Nordisk or Chirazyme® L-9 from Boehringer Mannheim. Celite, or other usual supports, can be used too.

In a first embodiment of the present invention, the acylation of flavonoid is carried out by conventional procedures. For example, the acylating agent can be an acyl halide, preferably acyl chloride. Examples of C₂-C₁₈ aliphatic acyl group can be, in the context of this invention, acetyl, propionyl, butyryl, valeryl, hexanoyl, heptanoyl, octanoyl, nonanoyl, decanoyl, lauroyl, myristoyl, palmitoyl, heptadecanoyl, oleoyl, stearoyl. Palmitoyl is preferred.

The next step is an alcoholysis carried out in organic solvent using an aliphatic alcohol possessing from 1 to 8 carbon atoms, as above disclosed.

Examples of this aliphatic alcohol are methanol, ethanol, propanol, isopropanol, n-butanol, t-butanol, amyl alcohol, n-hexanol, n-octanol. n-Butanol is preferred. Examples of polyols are glycerine and glycols.

The product of interest, namely the flavonoid 3-monoester, is recovered from the reaction mixture by conventional procedures, well known to the person skilled in the art. By suitably selecting the acyl group and the aliphatic alcohol, the desired product can be recovered by cooling the reaction mixture.

The process according to the present invention has produced 3-monoesters of quercetin, as shown in formula 1: 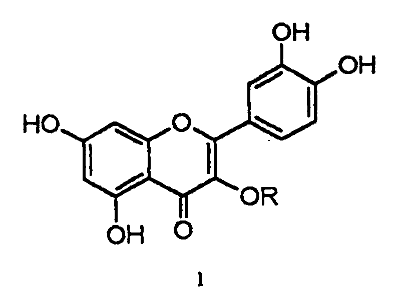 in which R is an acyl group of 3-18 carbon atoms.

Of galangin, as shown in formula 2: 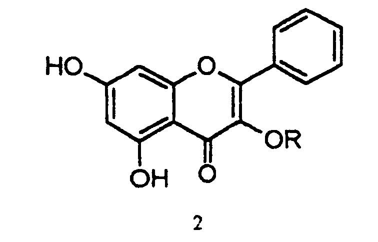 in which R is an acyl group of 3-18 carbon atoms.

Of morin, as shown in formula 3: 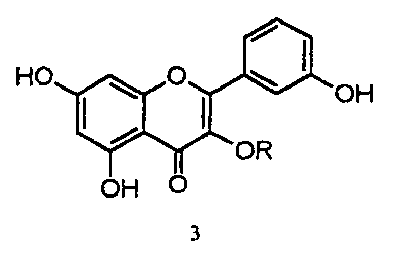 in which R is an acyl group of 2-18 carbon atoms.

Of fisetin, as shown in formula 4: 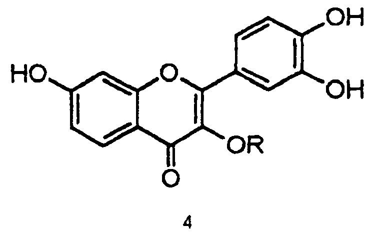 in which R is an acyl group of 2-18 carbon atoms.

Of kaempferol, as shown in formula 5: 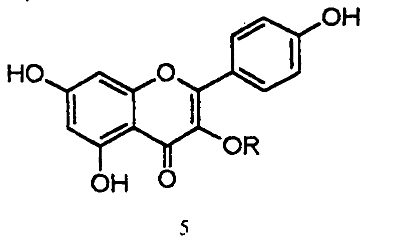 in which R is an acyl group of 2-18 carbon atoms.

Of kaempferide, as shown in formula 6: 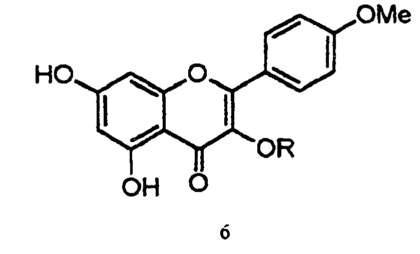 in which R is an acyl group of 2-18 carbon atoms.

Of (+)-catechin, as shown in formula 7: 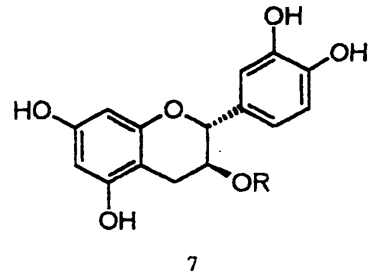 in which R is an acyl group of 2-18 carbon atoms.

Of (-)-catechin, as shown in formula 8: 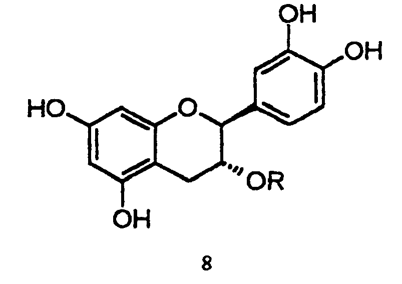 in which R is an acyl group of 2-18 carbon atoms.

Of (-)-epicatechin, as shown in formula 9: 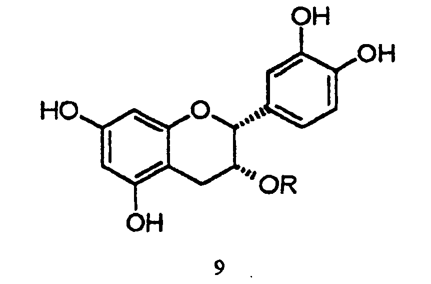 in which R is an acyl group of 2-18 carbon atoms.

Of (+)-epicatechin, as shown in formula 10: 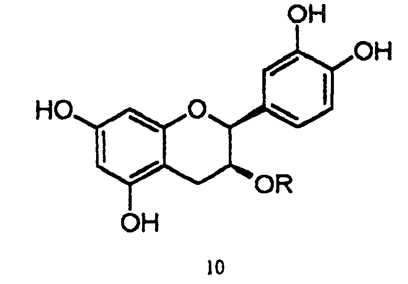 in which R is an acyl group of 2-18 carbon atoms.

These compounds are useful as antioxidants.

To provide an example of the experimental process, the specific case of quercetin is reported here.

Quercetin (1) is esterified by a conventional chemical process using an aliphatic acyl group having 1-18 carbon atoms, to give the corresponding pentacyl derivative (step A). These esters are subjected to alcoholysis with an aliphatic alcohol, having from 1 to 8 carbon atoms, in the presence of *Mucor miehei* lipases (Lipozyme® IM, Chirazyme® L-9, or adsorbed on inert support) using tert-butyl methyl ether as the solvent (step B).

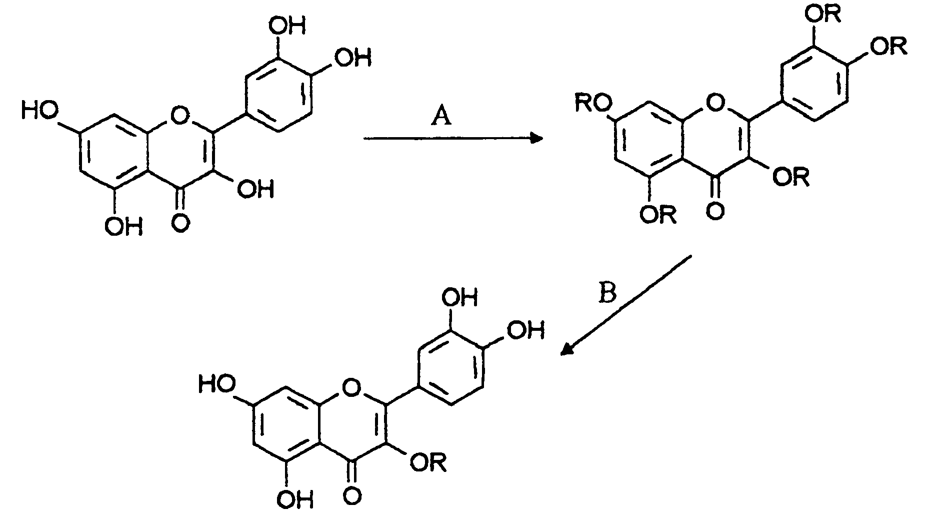

Alcoholysis leads to monoesters, in which all the phenolic hydroxyl functions are free, whereas the alcoholic group, at position C-3, remains esterified. It should be noted that the ester undergoing alcoholysis may have the hydroxyl groups on the A or B ring partially esterified. This is achieved by esterification with insufficient amounts of acylating agent or by a conventional protection process. Moreover, it is possible to use mixed esters in which hydroxyls in different positions are esterified by different acyls. In this way a number of advantages are realised. For example, alcoholysis of partial esters occurs faster. In the case of the mixed esters, if the desired 3-monoester possesses a long chain acyl group, it is advantageous to have all the other hydroxyls esterified with short chain acid groups, thus facilitating the conditions of the final alcoholysis.

The *Mucor miehei* lipase employed in immobilised form such as Lipozyme IM retains its activity throughout at least 10 cycles of use.

The use of lipases from porcine pancreas, Pseudomonas cepacia, Chromobacterium viscosum, Candida cylindracea, Candida antarctica, Aspergillus niger, Rhizopus niveus, Rhizopus javanicus, Rhizopus delemar, Candida lipolitica, Penicillium roqueforti, Penicillium cyclopium and esterase from porcine liver give scarce conversion or do not catalyse the alcoholysis at all.

The following example further illustrates the invention.

### Example:

Quercetin (1 g, 3.3 mmol) was dissolved in t-butyl methyl ether (50 mL) containing triethylamine (5 ml) and palmitoyl chloride (5.2 ml 7.2 mmol) was added at room temperature. After 12 hours the solution was acidified with dil. H₂SO₄ and extracted with CH₂Cl₂ three times. The obtained organic phase was taken to dryness to furnish 4.75 grams of pentapalmitoyl quercetin (96% yield).

Lipozyme IM (2.5 grams) was added to a solution of tert-butyl methyl ether (250 ml) containing pentapalmitoyl quercetine (3 g, 2.9 mmol) and n-butanol (1 1, 10.9 mmol) and the suspension was shaken (300 rpm) at 45°C. After 5 days the reaction was stopped and the enzyme filtered off. The solvent was removed under vacuum and the residue added to hexane (200ml). On cooling the solution, 1 g of 3-O-palmitoylquercetin (92% yield) was obtained as a low-melting solid.
¹H-nmr: ¹HNMR (CD₃Cl₃): δ 0.89 (bt, -CH₃), 1.30 (bs,-(CH₂)₁₃-), 1.73 (bt, -CH₂CO-), 6.31 (d, J= 2.2 Hz, H6), 6.55 (d, J= 2.2 Hz, H8), 7.02 (d, J= 8.2 Hz, H5'), 7.40 (dd, J= 2.2, 8.2 Hz, H6'), 7.47 (d, J=2.2 Hz, H2').

In the same way the following quercetin 3-monoesters have been prepared.

| Acyl group | R | Substrate Quercetin Pentacylated | Product Quercetin 3-0-acylated |
|---|---|---|---|
| Acetyl | C₂H₃O | C₂₅H₂₀O₁₂ | C₁₇H₁₂O₈ |
| Propionyl | C₃H₅₀ | C₃₀H₃₀O₁₂ | C₁₈H₁₄O₈ |
| Butyryl | C₄H₇₀ | C₃₅H₄₀O₁₂ | C19H1608 |
| Valeryl | C₅H₉0 | C₄₀H₅₀O₁₂ | C₂₀H₁₈O₈ |
| Hexanoyl | C₆H₁₁O | C₄₅H₆₀O₁₂ | C₂₁H₂₀O₈ |
| Heptanoyl | C₇H₁₃O | C₅₀H₇₀O₁₂ | C22H2208 |
| Octanoyl | C₈H₁₅O | C₅₅H₈₀O₁₂ | C₂₃H₂₄O₈ |
| Nonanoyl | C₉H₁₇O | C₆₀H₉₀O₁₂ | C₂₄H₂₆O₈ |
| Decanoyl | C₁₀H₉O | C₆₅H₁₀₀O₁₂ | C₂₅H₂₈O₈ |
| Lauroyl | C₁₂H₂₃O | C₇₅H₁₂₀O₁₂ | C₂₇H₃₂O₈ |
| Miristoyl | C₁₄H₂₇O | C₈₅H₁₄₀O₁₂ | C₂₉H₃₆O₈ |
| Heptadecanoyl | C₁₇H₃₃O | C₁₀₀H₁₇₀O₁₂ | C₃₂H₄₂O₈ |
| Oleoyl | C₁₈H₃₃O | C₁₀₅H₁₇₀O₁₂ | C₃₃H₄₂O₈ |
| Stearoyl | C₁₈H₃₅O | C₁₀₅H₁₈₀O₁₂ | C₃₃H₄₄O₈ |

## Claims

1. A process for the preparation of 3- monoesters of flavonoids comprising the following steps:
a) chemical esterification of a flavonoid with an aliphatic acyl group having from 1 to 18 carbon atoms to give the corresponding peracetylated flavonoid, or, alternatively, partially acylated flavonoid;
b) subsequent alcoholysis with an aliphatic alcohol having from 1 to 8 carbon atoms, in the presence of lipase from *Mucor miehei* in an organic solvent.

2. A process according to claim 1, in which the flavonoid is selected from the group comprising quercetin, galangin, morin, fisetin, kampferolo, kampferide, (+)-catechin, (-)-catechin, (+)-epicatechin, (-)-epicatechin.

3. A process according to claims 1 or 2, in which the aliphatic acyl is selected from the group comprising acetyl, propionyl, butyryl, valeryl, hexanoyl, heptanoyl, octanoyl, nonanoyl, decanoyl, lauroyl, miristoyl, palmitoyl, heptadecanoyl, oleoyl, stearoyl.

4. A process according to anyone of claims 1-3, in which the lipase from *Mucor miehei* is supported.

5. A process according to claim 4, in which the lipase is supported on celite.

6. A process according to anyone of claims 1-5, in which the solvent is a halogenated aliphatic hydrocarbon, an aromatic hydrocarbon, an ether.

7. A process according to claim 6, in which the solvent is tert-butyl methyl ether.

8. A process according to anyone of claims 1-7, in which, in step a) the esterification is partial and is performed with insufficient acylating agent.

9. A process according to anyone of claims 1-7, in which, in step a) the esterification is partial and is performed by previously protecting the hydroxyl groups and subsequent deprotection.

10. A process according to anyone of claims 1-7, in which, in step a) the esterification is partial and is carried out in successive steps, with different acylating agents, to give mixed esters.

## Revendications

1. Procédé de préparation de 3-monoesters de flavonoïdes, comprenant les étapes suivantes :
a) estérification chimique d'un flavonoïde avec un groupe acyle aliphatique ayant de 1 à 18 atomes de carbone pour donner le flavonoïde peracétylé correspondant ou, en variante, un flavonoide partiellement acylé ;
b) alcoolyse ultérieure avec un alcool aliphatique ayant de 1 à 8 atomes de carbone, en présence de lipase issue de *Mucor miehei* dans un solvant organique.

2. Procédé selon la revendication 1, dans lequel le flavonoide est choisi dans le groupe comprenant la quercétine, la galangine, la morine, la fisétine, le kaempférol, le kaempféride, la (+)-catéchine, la (-)-catéchine, la (+)-épicatéchine, la (-)-épicatéchine.

3. Procédé selon la revendication 1 ou 2, dans lequel l'acyle aliphatique est choisi dans le groupe comprenant acétyle, propionyle, butyryle, valéryle, hexanoyle, heptanoyle, octanoyle, nonanoyle, décanoyle, lauroyle, myristoyle, palmitoyle, heptadécanoyle, oléoyle, stéaroyle.

4. Procédé selon l'une quelconque des revendications 1 à 3, dans lequel la lipase issue de *Mucor miehei* est sur un support.

5. Procédé selon la revendication 4, dans lequel la lipase est sur un support en célite.

6. Procédé selon l'une quelconque des revendications 1 à 5, dans lequel le solvant est un hydrocarbure aliphatique halogéné, un hydrocarbure aromatique, un éther.

7. Procédé selon la revendication 6, dans lequel le solvant est le tert-butyl méthyl éther.

8. Procédé selon l'une quelconque des revendications 1 à 7, dans lequel, à l'étape a), l'estérification est partielle et est réalisée avec un agent acylant en quantité insuffisante.

9. Procédé selon l'une quelconque des revendications 1 à 7, dans lequel, à l'étape a), l'estérification est partielle et est réalisée par protection préalable des groupes hydroxyle et déprotection ultérieure.

10. Procédé selon l'une quelconque des revendications 1 à 7, dans lequel, à l'étape a), l'estérification est partielle et est réalisée par étapes successives, avec des agents acylants différents, pour donner des esters mixtes.

## Patentansprüche

1. Verfahren zur Herstellung von 3-Monoestem von Flavonoiden umfassend die folgenden Schritte:
a) chemische Veresterung eines Flavonoids mit einer aliphatischen Acylgruppe mit 1 bis 18 Kohlenstoffatomen, um das entsprechende peracetylierte Flavonoid, oder alternativ, das teilweise acetylierte Flavonoid zu erhalten;
b) darauf folgende Alkoholyse mit einem aliphatischen Alkohol mit 1 bis 8 Kohlenstoffatomen, in Gegenwart von Lipase aus *Mucor miehei* in einem organischen Lösungsmittel.

2. Verfahren nach Anspruch 1, wobei das Flavonoid ausgewählt ist aus der Gruppe umfassend Quercetin, Galangin, Morin, Fisetin, Kämpferol, Kämpferid, (+)-Catechin, (-)-Catechin, (+)-Epicatechin, (-)-Epicatechin.

3. Verfahren nach Anspruch 1 oder 2, wobei das aliphatische Acyl ausgewählt ist aus der Gruppe umfassend Acetyl, Propionyl, Butyryl, Valeryl, Hexanoyl, Heptanoyl, Octanoyl, Nonanoyl, Decanoyl, Lauroyl, Myristoyl, Palmitoyl, Heptadecanoyl, Oleoyl, Stearoyl.

4. Verfahren nach einem der Ansprüche 1 bis 3, wobei die Lipase aus *Mucor miehei* auf einem Träger vorliegt.

5. Verfahren nach Anspruch 4, wobei die Lipase auf Celite als Träger vorliegt.

6. Verfahren nach einem der Ansprüche 1 bis 5, wobei das Lösungsmittel ein halogenierter aliphatischer Kohlenwasserstoff, ein aromatischer Kohlenwasserstoff oder ein Ether ist.

7. Verfahren nach Anspruch 6, wobei das Lösungsmittel tert.-Butylmethylether ist.

8. Verfahren nach einem der Ansprüche 1 bis 7, wobei in Schritt a) die Veresterung teilweise und mit nicht ausreichendem Acylierungsmittel erfolgt.

9. Verfahren nach einem der Ansprüche 1 bis 7, wobei in Schritt a) die Veresterung teilweise erfolgt und durch vorhergehendes Schützen der Hydroxylgruppen und anschließendes Entschützen ausgeführt wird.

10. Verfahren nach einem der Ansprüche 1 bis 7, wobei in Schritt a) die Veresterung teilweise erfolgt und in aufeinander folgenden Schritten mit unterschiedlichen Acylierungsmitteln durchgeführt wird, um gemischte Ester zu erhalten.
